Europäisches Patentamt

European Patent Office

Office européen des brevets

(11) Publication number: **0 320 934**
**A2**

## (12) EUROPEAN PATENT APPLICATION

(21) Application number: **88120993.6**

(22) Date of filing: **15.12.88**

(51) Int. Cl.4: **H04R 1/12 , A61B 19/08**

(30) Priority: **18.12.87 IT 2309687**

(43) Date of publication of application:
**21.06.89 Bulletin 89/25**

(84) Designated Contracting States:
**AT BE CH DE ES FR GB GR IT LI LU NL SE**

(71) Applicant: **Romagnoli, Guido**
**V.le Zara, 117**
**I-20159 Milano(IT)**

(72) Inventor: **Romagnoli, Guido**
**V.le Zara, 117**
**I-20159 Milano(IT)**

(74) Representative: **De Nova, Roberto et al**
**c/o Jacobacci-Casetta & Perani S.n.c. Via**
**Visconti di Modrone 7**
**I-20122 Milano(IT)**

(54) **An antibacterical protection device for telephone handsets.**

(57) An antibacterial protection device (1) for telephone handsets (A), which provides full guarantee of sanitary conditions in that it prevents any contact of the ear and mouth with the speak-in and listen-to parts (B,C) of the handset A), comprises a thin membrane (2) having a predetermined surface area and bearing on one face (6) thereof an added adhesive material (7) for releasably applying the membrane (2) to the speak-in and listen-to parts (B,C) of the handset (A).

Fig-6

EP 0 320 934 A2

This invention relates to an antibacterial protection device for telephone handsets.

The need to use the telephone, and in particular public telephone sets, exposes the user to a risk of infection from bacteria which may have been transferred to the handset and found there suitable conditions of humidity and temperature for their subsistence and growth.

Survey work carried out on the handsets of public telephones installed at points of dense traffic has revealed that bacteria and mycetes nest therein in thousands, among which staphylococci responsible for skin infections, pharyngitis, abscesses, etc.

It may be appreciated, therefore, that public telephones are not at all safe to use and leave much to be desired from the sanitary standpoint.

It may, in fact, suffice to consider that the contact of the handset with the user's ear is unavoidable in practice, and that even incidental contact with one's mouth is not to be ruled out entirely. On occasions, moreover, in order to free one's hand, one is forced to hold the handset in the clasp between one's shoulder and face, thus increasing the risk of such easily affected parts as the ear and mouth coming into contact with the infected areas of the handset.

Disinfection procedures have been proposed which are performed by specialized concerns using suitable products to kill the bacterial charge. Such procedures, while being in many ways satisfactory, still has the drawback that they must be applied periodically. In fact, already after a fortnight period, the effect of the disinfection treatment is exhausted and the bacterial contamination condition restored, and with it, the user's health endangering situation. Furthermore, the circumstance is not to be underestimated of some individuals being allergic to the products used for the disinfection treatment.

The problem underlying this invention is to provide an antibacterial protection device which has such structural and performance characteristics as to permit operation of public telephones in a sanitarily safe condition.

This problem is solved by a device as indicated being characterized in that it comprises a thin membrane having a predetermined surface area, and releasable attachment means for applying the membrane to the telephone handset.

Further features and the advantages of a device according to the invention will become apparent from the following detailed description of a preferred embodiment thereof, to be taken by way of illustration and not of limitation in conjunction with the accompanying drawings, where:

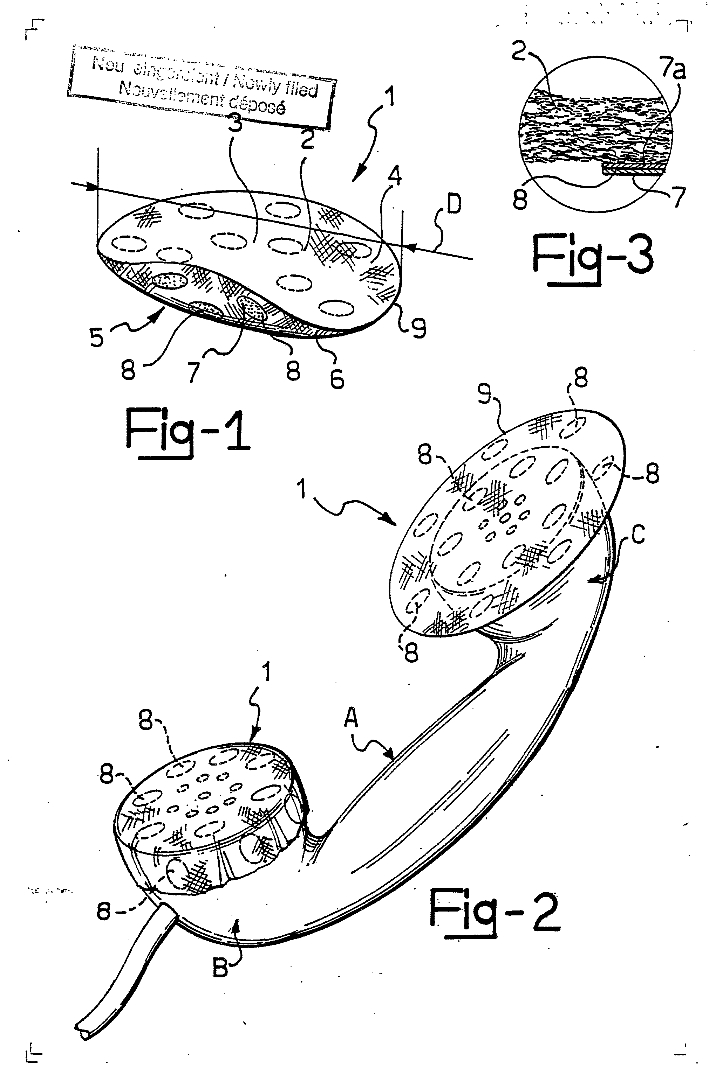

Figure 1 is a perspective view of a device according to the invention;

Figure 2 is a perspective view of a telephone handset provided, at both its "talk-in" and "listen-to" ends, with respective devices according to the invention;

Figure 3 is a sectional view showing to a much enlarged scale the device of Figure 1; and

Figures 4, 5 and 6 show similar views of another embodiment of the device according to the invention.

With reference to the drawing views, the numeral 1 generally designates an antibacterial protection device according to the invention. The device 1 comprises a thin membrane 2 having a predetermined surface area, e.g. a circular surface with a diameter D of approximately 7 cm, a central portion 3, and a peripheral portion 4. The membrane 2 is made of paper.

A crinkled paper has shown to yield best results. That paper is formed from a paste consisting of cellulose obtained from chemically bleached pulp releasing no offensive odours in either the dry or wet conditions, and containing no regenerated paper other than manufacturing waste from the same batch.

The paper contains no such foreign chemicals as dyes, toxic matter, or chemical disinfectants, which might be released in use, and would release no lint or fiber matter under conditions of normal use.

This paper has the following characteristics:

| Basis weight | 56 g/m² min. |
|---|---|
| Extraction water pH | 5.0 : 8.0 |
| Chlorides, % | .05 |
| Sulphates, % | .25 |
| Fluorescence | 5 x 1 mm/100 cm² |
| Elongation, lengthwise | 10% min. |
| Elongation, crosswise | 2% min. |
| Ultimate strength, lengthwise, dry | N/15 mm 20 min. |
| Ultimate strength, crosswise, dry | N/15 mm 10 min. |
| Ultimate strength, lengthwise, wet | N/15 mm 5 min. |
| Ultimate strength, crosswise, wet | N/15 mm 4 min. |
| Water repellency, sec.s | 18 min. |
| Methylene blue penetration | 20 max. |
| Bursting strength, wet | 35 kPa |
| Bursting strength, dry | 110 kPa |
| Permeability to air | 3.9 microns/Pa.s min. |
| Surface absorbency (Cobb) | 20 g/m² max. |

The paper surface is slightly embossed to promote release.

It is commercially available, e.g. under the trade name of "Steri-Green" from 3M.

For detachably applying the membrane to a handset A, at either the so-called "speak-in" end B or so-called "listen-to" end C thereof, the device 1 is provided with attachment means, indicated at 5.

The attachment means 5 comprise an added adhesive material 7 which is applied over a face 6 of the membrane.

Preferably, the surface of the membrane 2 would be applied, at the location of the added adhesive material 7, a neutral primer 7a effective to prevent penetration of the adhesive material through the paper.

The added adhesive material 7 is embodied in actual practice by a plurality of sticking spots, collectively designated 8, which are distributed at regular intervals across the face 6 in the peripheral portion 4 of the membrane.

The sticking spots 8 are circular in shape and have a diameter dimension of a few millimeters, being set approximately one centimeter apart to ensure full adhesion of the membrane on the handset.

Advantageously, the membrane 2 has a rim 9 intended to stand a few millimeters proud of the "speak-in" and "listen-to" ends of the handset and for folding over in order to fit the device closest to the handset.

To apply this device to a telephone handset, it will be sufficient to so lay the membrane over the handset as to bring the sticking spots into contact with the "speak-in" or "listen-to" end surface along the peripheral portions thereof.

The handset is thus made ready for use.

After the call has been made, the device can be readily removed by grasping the membrane at its outer rim, peeling it off for subsequent disposal.

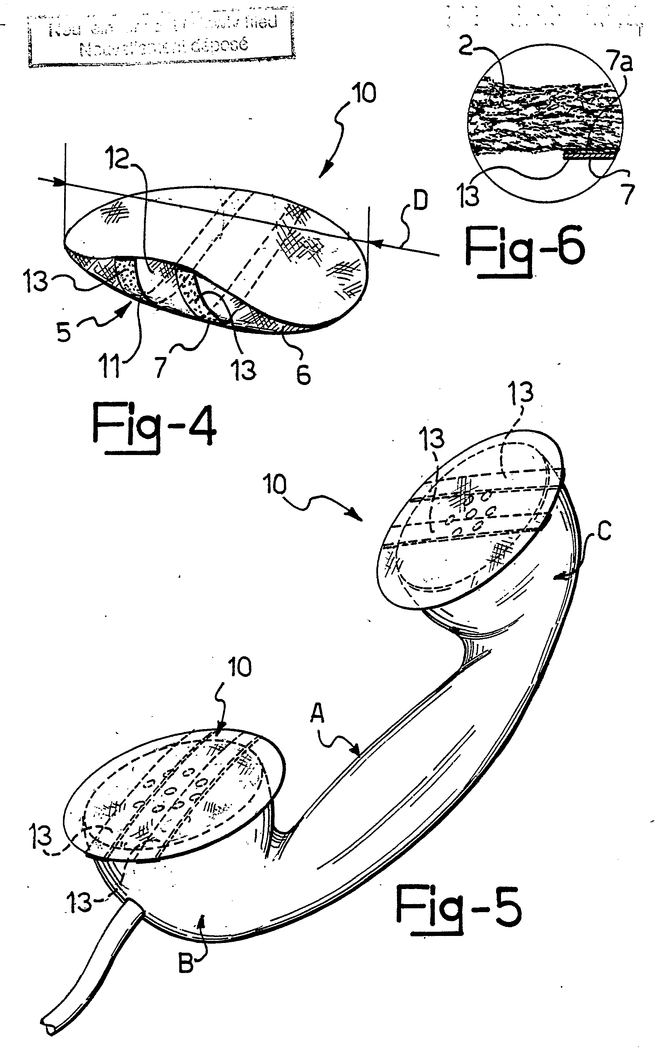

Another embodiment of a device 10 according to the invention is shown in Figures 4, 5 and 6. Throughout these views, parts of the device 10 having similar constructions and operating in the same way as those of the device 1 are denoted by the same references and will be no further described.

The added adhesive material 7 is here an adhesive strip 10 which extends across a diametrical portion 12 of the membrane 2. The adhesive strip 11 is preferably composed of two parallel continuous bands 13 which are a few millimeters wide and set approximately one centimeter apart, to ensure close adhesion of the membrane on the handset and easy removal of the membrane after use.

A major advantage of the inventive device is that it is a guarantee of a fully sanitary condition of use, inasmuch as it is capable of preventing any contact of the ear and mouth with the telephone handset in use.

The paper membrane has shown to constitute a filter effective to provide a barrier to bacteria passage.

Furthermore, no risk is run by individual perceptive of allergies using a device according to the invention.

In any case, the absence of foreign chemicals excludes all possibilities for contamination, from either the environmental or sanitary standpoint, such as originating from carcinogenic agents.

The device of this invention, moreover, can retain its effectiveness protection-wise unaltered over time.

A further advantage of the device according to this invention is that it is easy to use, since it can be carried along without adding appreciably in the way of bulk and weight, and readily applied to a telephone handset and just as readily removed and thrown away.

EP 0 320 934 A2

Actual tests have also shown that it affects the telephone performance in no detectable way and affords perfect listening conditions for both conversations.

The device construction enables it to be used with traditional design handsets as well as most up-to-date handsets irrespective of their design.

In addition, the inventive device has a simple construction, which is no least advantage with a device which is to be made in very large volumes and fully disposable after use. effectiveness protection-wise unaltered over time.

It is also suitable for packaging in light and compact packages, still containing a reasonable supply of it. The device may be distributed through manually or automatically operated vendor machines installed at public telephone sites.

Finally, the device according to the invention is also favorable from the environmental standpoint, it being formed from fully biodegradable materials.

It should be noted that the inventive device lends itself to accommodating on its surface appropriate inscriptions providing instructions on the manner of using it, as well as, of course, any desired inscriptions, such as an indication of its manufacturer and/or advertisements.

Lastly, it is to be expected that the cost of the device, however small, can be fully recovered through a reduced cost for social medical assistance to be achieved by virtue of the improved containment of ailments afforded by this device.

Understandably, the device disclosed hereinabove may be in many ways altered and modified by a skilled person in the art for the purpose of meeting specific contingent demands, without departing from the true scope of the invention as set forth in the appended claims.

**Claims**

1. An antibacterial protection device (1) for telephone handsets (A), characterized in that it comprises a thin membrane (2) having a predetermined surface area, and releasable attachment means (5) for applying the membrane (2) to the telephone handset (A).

2. A device according to Claim 1, characterized in that said attachment means (5) comprise an added adhesive material (7) applied over one face (6) of the membrane (2).

3. A device according to Claim 2, characterized in that the membrane surface is applied a neutral primer (7a) at the location of the added adhesive material (7).

4. A device according to Claim 3, characterized in that said added adhesive material (7) is embodied by a plurality of sticking spots (8) distributed at regular intervals across a peripheral portion (4) of the membrane (2).

5. A device according to Claim 3, characterized in that said added adhesive material is embodied by an adhesive strip (11) extending across a diametrical portion (12) of the membrane (2).

6. A device according to Claim 5, characterized in that said adhesive strip(11) comprises two parallel continuous adhesive bands(13).

7. A device according to Claim 1, characterized in that said membrane (2) is made of paper.

8. A device according to Claim 7, characterized in that said paper is crinkled paper.

9. A device according to Claim 8, characterized in that said paper is formed from a paste consisting of cellulose from chemically bleached pulp releasing no offensive odours either in the dry or wet condition, and containing neither regenerated paper other than manufacturing waste from the same batch nor such foreign chemicals as dyes or toxic matter or disinfectants as may be released in use.

10. A device according to Claim 9, characterized in that said paper has the following characteristics:

4

| | |
|---|---|
| Basis weight | 56 g/m$^2$ min. |
| Extraction water pH | 5.0 : 8.0 |
| Chlorides, % | .05 |
| Sulphates, % | .25 |
| Fluorescence | 5 x 1 mm/100 cm$^2$ |
| Elongation, lengthwise | 10% min. |
| Elongation, crosswise | 2% min. |
| Ultimate strength, lengthwise, dry | N/15 mm 20 min. |
| Ultimate strength, crosswise, dry | N/15 mm 10 min. |
| Ultimate strength, lengthwise, wet | N/15 mm 5 min. |
| Ultimate strength, crosswise, wet | N/15 mm 4 min. |
| Water repellency, sec.s | 18 min. |
| Methylene blue penetration | 20 max. |
| Bursting strength, wet | 35 kPa |
| Bursting strength, dry | 110 kPa |
| Permeability to air | 3.9 microns/Pa.s min. |
| Surface absorbency (Cobb) | 20 g/m$^2$ max. |

**Fig-1**

**Fig-3**

**Fig-2**

10

12

D

13

5

11  7  13  6

**Fig-4**

2  7a

13  7

**Fig-6**

13  13

10

C

10

A

13

13

B

**Fig-5**